# EUROPEAN PATENT APPLICATION

(11) **EP 2 229 880 A1**
(43) Date of publication of application: **22.09.2010**
(21) Application number: 09155536.7
(22) Date of filing: 18.03.2009
(51) Int. Cl.: A61B 5/024, A61B 5/11

(54) **Headband integrated monitoring unit using an accelerometer**

(71) Applicant: CSEM Centre Suisse d'Electronique et de Microtechnique SA, 2002 Neuchâtel (CH)
(72) Inventor: Correvon, Marc, 2087, Cornaux (CH); Ridolfi, Andrea, 3008, Bern (CH); Rossini, Leopoldo, 6528, Camorino (CH); Vetter, Rolf, 1116, Cottens (CH)
(74) Representative: P&TS Patents & Technology Surveys SA

(57) **Abstract**

A monitoring device (1) worn by a user comprising a heart rate sensor (3) for delivering a sensor cardiovascular (CV) signal, a signal processing device (5) for correcting artifacts due to user's motion in said sensor CV signal, and a motion sensor (4) delivering a motion reference signal; the motion sensor (4) having a main function for determining and outputting a signal representing the level of activity of the user, and an auxiliary function for controlling said signal processing device (5). The monitoring device may be disposed in a headband (2) worn on the user's head. The monitoring device may comprise the monitoring of other physiological signals such as SPO₂.

## Description

### Field of the invention

The present invention concerns a robust monitoring device for monitoring the heart rate, SPO₂, running speed and/or activity of a user, based on a sensor unit.

### Description of related art

It is valuable for an exerciser to know his/her heart rate and speed information during exercise or an activity. Current heart rate and speed monitor is usually based on an electrocardiogram (ECG) signal-based sensor fixed on a chest belt for heart rate measurement, and acceleration sensor based or GPS technology. A foot pad embedded with an accelerometer can also be used to measure speed and a wrist watch to display heart rate and speed information received separately from the chest belt and foot pad. However, this solution is bulky and the supplementary weight added to the runner's shoe increases the runner oxygen consumption and diminishes its performance by about the same ratio.

US2007062279 discloses a system for measuring gait kinematics information during exercise of a subject comprising a fixing member, such as a chest belt, fixed onto the trunk of the subject; an acceleration sensor attached to the belt for sensing the vertical acceleration of the trunk of the subject; and a microprocessor coupled to the acceleration sensor. The microprocessor is adapted to receive the vertical acceleration data from the acceleration sensor, and to compute and derive the speed and distance traversed by the subject, and to communicate the speed and distance information.

In a preferred embodiment, an ECG-based heart-rate monitoring device is also provided onto said belt and coupled to said microprocessor. This solution also has the disadvantage of being bulky.

A drawback with heart rate measurements on a user in movement is, indeed, the corruption of the heart rate signal by user movements, so-called motion artifacts. These artifacts lead to erroneous interpretation of heart rate signals and degrade the accuracy and reliability of the estimation of cardiovascular parameters.

In W02007072239, a watch-like device for monitoring a user's heart rate comprises two electrodes placed along the surface of a wrist strap, in contact with the user's skin, in order to measure changes in an electrocardiogram (ECG) signal. The device further comprises an accelerator for determining an acceleration of the user's arm and compensate for the arm movement in the heart rate signal. The compensated ECG signal may be used as a parameter in computer games. Here, the accelerator signal itself is only used for correcting movement artifacts in the heart rate signal and the speed or level of activity of the user is not estimated.

A portable equipment, such as a walkman, for example, or a hearing aid, including a photoplethysmography (PPG) -based heart rate measuring device and a sound reproduction unit is disclosed in US2003233051 by the present applicant. The equipment includes means for supplying a signal representative of the sound reproduction and sound information to a sound transducer. The measuring device and sound transducer are mounted in an assembly adapted to be fixed to an ear of a wearer. The equipment further includes an accelerometric device delivering motion signals representative of the motion of a wearer, and signal processing means using the motion signals for removing artifacts due to motion in the signal representative of the heart rate.

The processing of motion artifacts in PPG measured signals has also been addressed by the present applicant in patent application EP1297784. The application discloses a portable pulse rate detecting device which is adapted to be worn on the wrist of a user, and a signal enhancement method that exploits the information contained in a motion reference signal generated by a three-dimensional accelerometer, also placed on the wrist, in order to obtain a robust PPG heart rate estimation, even under intense physical activity.

### Brief summary of the invention

An object of the invention is therefore to propose a new system and method which overcomes at least some limitations of the prior art.

According to the invention, these objectives are achieved by means of a system and method comprising the features of the independent claims, preferred embodiments being indicated in the dependent claims and in the description.

These objectives are also achieved by a monitoring device worn by a user comprising a heart rate sensor for delivering a sensor cardiovascular (CV) signal, a signal processing device for correcting artifacts due to user's motion in said sensor CV signal, and a motion sensor delivering a motion reference signal; the motion sensor being characterized by having : a main function for determining and outputting a signal representing the level of activity of the user, and an auxiliary function for controlling said signal processing device.

In an embodiment of the invention, the motion reference signal delivered by the motion sensor is exploited in the signal processing device to provide a robust heart rate signal corrected for the movement artifacts from the sensor CV signal.

In another embodiment of the invention, the motion reference signal delivered by the motion sensor is exploited in the signal processing device to provide an activity level reference signal representative of the level of activity of the user.

In yet another embodiment of the invention, the robust heart rate signal is used in combination with the activity level reference signal to assess and monitor the fitness of the user or the efficacy of a sportive activity during an activity process.

In yet another embodiment of the invention, the signal representing the level of activity corresponds to the speed of the user.

In yet another embodiment of the invention, the monitoring device further comprises the monitoring of oxygen saturation of arterial blood (SP02) by measuring a SP02 signal using the heart rate sensor. The motion reference signal can be used to obtain a SP02 signal free from motion artifacts.

In a preferred embodiment of the invention, the heart rate sensor and the motion sensor are disposed in a headband worn on the user's head.

The monitoring device of the invention is compact, simple and comfortable to wear by a user for extended time periods. Using the monitoring device of the invention, a motion reference signal with a robust heart rate signal, free from motion artifacts, can be combined in order to reliably assess and monitor the fitness of the user during an activity process, for example, during a sportive or fitness activity.

Moreover, the PPG-based heart rate sensor can be used for the measurement of several physiological parameters other than the CV signal, such as SPO₂, arterial blood pressure, breathing, etc. The monitoring device can also comprise additional sensors enabling the measurement of many other parameters such as the temperature and the electrodermal activity; all sensors being integrated on the headband.

### Brief Description of the Drawings

The invention will be better understood with the aid of the description of an embodiment given by way of example and illustrated by the figures, in which:
Fig. 1 illustrates schematically the monitoring device according to an embodiment of the invention; and
Fig. 2 shows the monitoring device according to another embodiment of the invention.

### Detailed Description of possible embodiments of the Invention

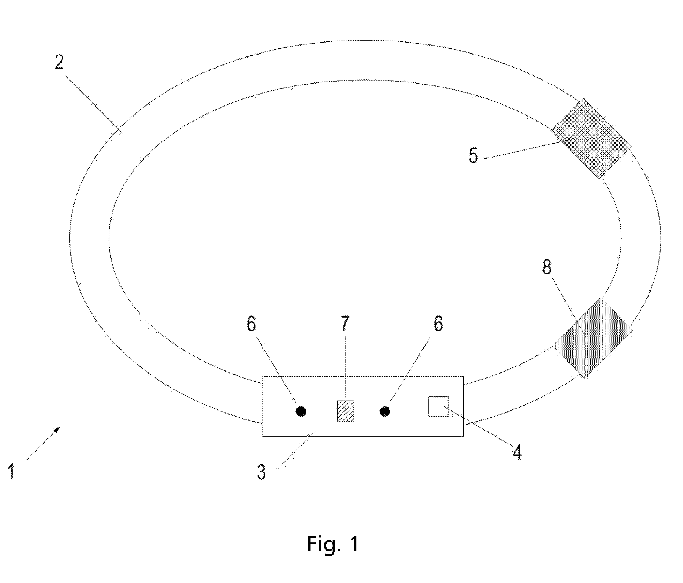

Fig. 1 illustrates schematically a monitoring device 1 according to an embodiment of the invention comprising a heart rate sensor 3, a motion sensor 4, and a signal processing module 5. In the example of Fig.1, the monitoring device 1 is disposed in a headband 2 worn on the user's head.

In the present embodiment, the heart rate sensor 3 is based on an electro-optic technique, such as photoplethysmography (PPG) described in documents US7018338 and US717560. In the PPG technique, the heart pulse wave caused by the periodic pulsations of arterial blood volume is measured by the changing optical absorption of radiant energy. The PPG based heart rate sensor 3 comprises at least one emitter 6, preferably an infrared light emitting device (LED), and at least one receiver 7, preferably a photodiode, for detecting the intensity of the radiant energy after propagation through the user's skin.

The emitter 6 and the receiver 7 can be placed along the headband 2, on the side which is in contact with the user's skin, preferably at locations in the vicinity of a superficial artery such as, for example, the superficial temporal artery in the temporal region or the supraorbital or zygomatico-orbital artery in the front head area. In this configuration, light from the optical emitter 6 propagates through one of the artery above and is reflected towards the optical radiation receiver 7. Other locations of the emitter 6 and the receiver 7 along the headband are however also possible.

In the example of Fig. 1, the emitter 6 comprises a pair LEDs disposed on each side of the photodiode used as receiver 7, each LED 6 being at a determined distance from the photodiode 7, for example, about 10 mm. Other possible arrangements of the emitter and receiver can comprise one or several LED and photodiodes respectively. The spatial arrangement of the photodiodes should be selected in a way to provide sufficient spatial diversity in order to remove artifacts due to tissue inhomogeneities.

As shown in the example of Fig. 1, the motion sensor 4 is preferably placed within the heart rate sensor 3, possibly forming an integrated sensor comprising the heart rate sensor 3 and the motion sensor 4. In this configuration, the signal measured from the motion sensor 4 is better correlated with the measured heart rate sensor 3 signal than if the motion sensor 4 is placed further from the heart rate sensor 3.

The motion sensor 4 is preferably a MEMS-based two-dimensional or three accelerometer composed of two accelerometers disposed along two orthogonal directions and providing acceleration signals corresponding to acceleration components operatively aligned along a respective orthogonal axis. It will however be appreciated that other types of accelerometers or motion detecting devices can be used provided they deliver a reliable measure of motion. For example, the motion sensor 4 could be a gyro-sensor of any suitable technology incorporating a one or two dimensional accelerometer, or a rotating or vibrating element. The motion sensor 4 can also be a three-dimensional accelerometer, for example, such as the one manufactured by the company Colibrys S. A. under reference MS 6100.

The electrical cardiovascular (CV) signal delivered by the receiver 7 and acceleration signals delivered by the accelerometers of the motion sensor 4 are transferred to a signal acquisition module 8, placed in the headband 2 and comprising, for example, an analog to digital (A/D) converter and signal filtering and shaping means. The acquired CV and acceleration signals are then transferred to the signal processing module 5. A battery that can also be placed in the headband 2 powers the monitoring device 1. The processing module 5 can be an adequately programmed digital signal processor or DSP or a general purpose microcontroller.

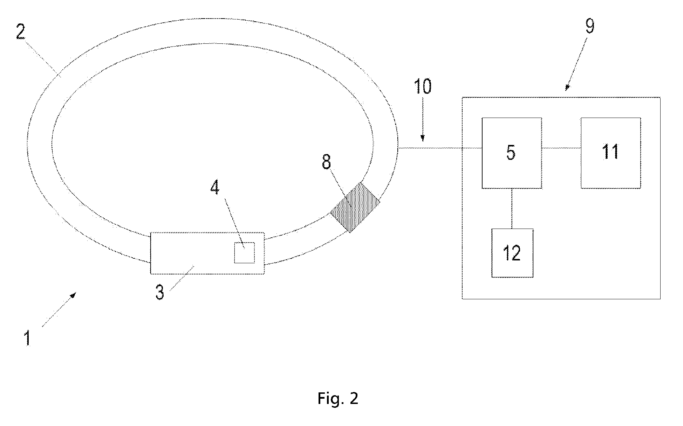

Fig. 2 shows another embodiment of the invention, where the signal processing module 5 is placed within an external control device 9. The control device 9 can be a media player such as the digital Apple iPod media player or any other handheld computing device such as a cellular telephone, personal digital assistant or other devices having an operating system. Other portable devices including compact disk (CD) or versatile disks (DVD) players, radio receiver, etc., may also be used as well as a control device dedicated to the sole sensing purpose. The control device 9 can also be a wristwatch, similarly to some of the heart monitoring systems proposed by Polar Electro Oy. In this configuration, the acquired CV and/or acceleration signals outputted from the acquisition module 8 are transferred to the signal processing module 5 through a cable 10 that can be connected to the control device 9 through a USB connector (not shown) or any other type of connector. In this configuration, the monitoring device 1 can be powered by a battery (not shown) contained in the control device 9.

Alternatively, the acquired CV and/or acceleration signals are transferred to the signal processing device 5 in a wireless fashion. In that case, the acquired CV signal is protected from the acquired acceleration signal in order to avoid possible perturbations of one type of signal from the other.

In yet another embodiment not represented, the acquisition module 8 can also be placed in the control device 9 or, alternatively, the functionalities of the acquisition module 8 can be performed by the signal processing device 5 located within the control device 9.

In yet another embodiment, the signal processing module 5 can be installed to the existing media player, handheld computing device or any other portable device; the processor of the media player, handheld computing device or portable device being used for the acquired sensor CV signal processing.

In yet another embodiment, the heart rate and/or motion sensor 3, 4 may include an embedded signal processor (not shown) or other type of dedicated processors for performing any desired processing of the measured heart rate or acceleration signals output from the heart rate sensor 3 and/or accelerometers, respectively, prior to outputting signals to the signal processing device 5.

Other configurations are also possible, for example, a digital signal processor or DSP can be used in conjunction with signal filtration, A/D conversion, and other signal conditioning/processing within the headband 2 or control device 9 in order to extract useful heart rate information from the measured CV signal and/or acceleration signals. Alternatively, the functionalities of the signal acquisition module 8 and signal processing module 5 can be performed by using one or more application-specific integrated circuits (ASICs).

It is understood that the present invention is not limited to the exemplary embodiments described above and other examples of implementations are also possible within the scope of the patent claims.

In another embodiment of the invention (not represented), the heart rate sensor 3, and possibly the motion sensor 4, are located in an ear located device, for example, earphones used with off-the-shelf walkmans, MP players, ipod, etc., comprising a hook adapted to hang behind the external ear of a user and a generally circular casing adapted to be placed inside or against the external ear (auricle) of a user, Here, the emitter 6 of the heart rate sensor 3 can be placed in the hook and the receiver 7 in the casing so that it can pick up the portion of the optical radiation emitted by the emitter 6 that has passed through the thickness of the external ear. Different placements of the emitter 6 and receiver 7 are also possible. For example, the emitter 6 and the receiver 7 can be placed along the hook, on the side which is in contact with the user's skin. The heart rate sensor 3 located in the ear located device can be linked with the headband with a cable or wirelessly.

Alternatively, the ear located device can be small earphones, placed outside the external auditory meatus such as the distinctive white earphones that are included with many portable music players, or placed inside the external auditory meatus, such as canalphones. Here, the optical emitter 6 and the optical receiver 7 can be placed on the surface of one of the earphones and the light emitted by the optical emitter 6 propagates through the tissue in the vicinity of the external auditory meatus and is reflected towards the optical receiver 7.

In yet another exemplary embodiment, the monitoring device 1 can be fitted to an eyeglass, the heart rate sensor 3 being placed on the eyeglass frame, for example, in the temple and/or nose regions while the motion sensor 4 can be placed on one or each eyeglass branch. Here, a display unit using liquid crystal or the like can be placed in front of the eyeglass in order to display information related to, for example, heart rate to the user.

In yet another embodiment of the invention (not represented), the heart rate sensor 3 is based on an ECG technique using at least two electric potential electrodes. Here, one electrode can be placed on the headband 2 worn on one user's head and the other electrode can be disposed on another strap (not shown) worn, for example, on the other user's wrist, or a chest belt, both electrodes being in contact with the user's skin. In another embodiment, both electrodes can be placed on the headband 2, in a similar fashion to the apparatus described in W02007/072239 in the case of a wristband. Flexible electrodes, for example such as described in US20060142654, can also be integrated into the headband and/or the strap tissue or into a garment fabric. The electrodes can be connected to a comparator (not shown) for determining a difference value between the measured electric potentials at the different electrode locations. In addition, the heart rate sensor 3 may comprise a signal acquisition module 8 outputting an acquired CV signal that is transferred to the signal processing device 5 through a cable 10 or wirelessly.

In yet another embodiment, the heart rate sensor 3 can be based on an impedance cardiography (ICG) technique or a combination of at least two of the electro-optic technique, ECG or ICG techniques.

The main function of the motion sensor 4 is to determine the level of activity of the user, for example, the speed of the user when running on foot. In the case of a two-dimensional accelerometer motion sensor 4, the accelerometer aligned along the vertical axis can provide an electrical acceleration signal representative of the vertical acceleration of the runner during the duration of at least one of the runner's strides. The other accelerometer can be used to measure the lateral component of the acceleration of the user in order to separate the speed contribution of the right foot from the left foot. Here, the placement of the accelerometers on the headband 2 approximates essentially gravity center based accelerometer measurement on the user.

The user speed determination is based on the fact that the vertical acquired acceleration signal changes sign at the moment at which the runner's leg lifts again after having touched the ground. The speed is determined by calculating the signal slope at the moment the acquired acceleration signal changes sign. The slope can be calculated for the right and left feet of the user and the runner speed can be determined for the two feet. Slope calculation and speed determination can be performed in the signal processing device 5 in order to provide a reliable activity level or speed reference signal. To this end, the signal processing device 5 can comprise an implemented algorithm and memory means for storing predetermined values.

Alternatively, the runner's speed can also be determined based on the acquired acceleration signal during the time period where the runner is in the air and the time period where the runner is touching the ground. The runner speed can also be determined by a combination of the two methods described above. A more detailed description of the method and device for determining the user's speed is given in patent application EP1913336 by the present applicant.

The acquired CV signal originating from the heart rate sensor 3 are enhanced using the signal processing module 5 and possibly a motion reference signal provided by the motion sensor 4, in order to correct the acquired CV signals for movement artifacts. The signal enhancement is performed using a method described in PCT application PCT/EP2007/063469 by the present applicant. The method assumes that under rhythmical movements, artifacts on the acquired CV signal appear as harmonics of the movement frequency. The method comprises the steps below.

In a first step, the fundamental movement frequency is extracted from the motion reference signal through one of the following technique: zero-crossing, parametric or non-parametric spectral estimation, autocorrelation, recurrence plots.

In a second step, the fundamental movement frequency extracted in the first step is used to enhance the acquired CV signal. Here, for example, a multiple notch filter can be employed to attenuate harmonics of movement contributions in the acquired CV signal. Alternatively, non-parametric enhancement can be used where the movement contribution is cancelled, for example, by putting high resolution restricted discrete Fourier transform estimations at zero from nearest lower minimum to nearest upper minimum at each harmonic of movement frequency.

In a third step, heart rate signals are extracted from the enhanced CV signal using a zero-crossing, parametric or non-parametric spectral estimation, autocorrelation or recurrence plot method.

In a possible further fourth step, the reliability of the heart rate signals is assessed in order to merge and/or discard heart rate signals based on a reliability indicator and provide a reliable robust heart rate signal corrected for motion artifacts.

The enhancement may also be performed by a subband approach. This may considerably increase the robustness of the subsequent heart rate estimation. In this case the acquired CV signal is filtered by typically three to four adjacent subband filters with a bandwidth of the fundamental movement frequency and located on harmonics of the movement frequency. Each subband filter is designed in such a way that the lower and upper corner frequency correspond to zeros of the given filter (as obtained for Tchebycheff class 2 filters or elliptic filters). The heart rate estimation may then be performed on each subband separately and the most reliable of all bands according to the reliability indexes may be retained.

In some conditions, the user's fundamental movement frequency may become synchronized with the user's heart rate. This may happen, for example, when the frequency of the steps of a jogger approaches its heart rate. In this case, the fundamental movement frequency of the motion reference signal overlaps completely the heart rate component of the CV signal and the extracted fundamental movement frequency can be used instead of the enhanced CV signal to estimate the user's heart rate signal. As soon as the user's movement is no more in phase with its heart rate, the user's heart rate signal can be again estimated using the enhanced CV signal as in the method described above.

In an aspect of the invention, a computer program product configured to be operable on the signal processing device 5 is provided in order to carry out the processing of the acquired signals described above, *i.e.,* to correct the acquired signals for artifacts, and perform the speed or level of activity determination. The software product can be downloaded in a memory 11 associated with the signal processing device 5. The downloading operation can be performed using a storage reader device (not shown), such as a CD or DVD reader, a USB memory key or a flash memory, etc., integrated on the processing device 5 or control device 9, or as an removable storage reader device (not shown), connected to the control device 9 through a USB connector or any other type of connector. The downloading operation can also be performed in a wireless fashion.

In a preferred embodiment of the invention, the artifact correction, speed or activity determination and signal display are performed in real time on the stream of data delivered by the sensors. Post processing performed afterward, for example on a PC, is also possible.

The monitoring device 1 may further comprise an output device 12 for outputting an indication of the heart rate signal and/or determined activity level reference signal in the form of an optical, audible signal, or other sensorial signal. Such means could be a LED, a display, a buzzer, a vibrating device or any other suitable device adapted for transmitting information representative of the determined heart rate and/or the user speed or level of activity.

The outputted signal may be an alarm signal, such as a beeping sound or a flashing light, when the estimated heart rate value, and/or the speed or activity level of the user, reaches a determined threshold, which could be either a low or high threshold or both. In the case the signal processing module 5 is placed within a media player, the outputted sound signal can also be superimposed on the sound signals produced by the media player and make it audible to the user, for example, through a sound transducer of an ear located device.

Alternatively, the output device 12 can be any known display means such as the LCD display unit able to represent the heart rate and/or speed or activity level values. The output device 12 can also be LCD display of the iPod media player, cellular telephone, etc., or a visual display of wristwatch.

In yet another embodiment of the invention (not represented), the heart rate signal and the speed or activity level reference signal are transferred to an external console, for example, through a wireless transfer device 6 such as Bluetooth, 802.11 UWB (ultra wide band), infrared, and the like, or through a wired connection. The console can be associated with a running belt, a cycling apparatus, or used in combination with an electronic game or fitness activity process.

The monitoring device 1 of the invention allows the user to combine a motion reference signal with a robust heart rate signal, free from motion artifacts. The two signals can be advantageously combined, for example, in order to assess and monitor the fitness of the user during an activity process, for example, during a sportive or fitness activity. For example, the speed of a runner, determined as described above, can be combined with the user's robust heart rate signal, free from motion artifacts, allowing the runner to reliably monitor its fitness during the exercise. The acquired acceleration signal can also be used in a pedometer to determine and output data representing the locomotion of the individual, or can be used to translate the user's movements, such as the number of steps, jumps, jogging, biking or any other movements, in a parameter in an activity involving physical exercising, such as a fitness activity or an electronic game.

The monitoring device 1 of the invention can also advantageously be used for the monitor of the efficacy of a sportive activity. Here, the efficacy is determined by activity level corresponding to a constant speed and a minimal acceleration of the gravity center, determined, for example, by a method such as the one described in patent EP1586353 by the present applicant. The method comprises the steps of extracting at least an attribute from the acquired acceleration signal in order to capture the acceleration associated with a user movement, and comparing this attribute with an average value of the same attribute, obtained for a reference population, corresponding to a percentage of the maximum heart rate provided by the artifact-free heart rate signal.

In another exemplary embodiment, the monitoring device 1 also comprises the monitoring of other vital or physiological signals such as oxygen saturation of arterial blood (SPO₂). Here, the PPG-based heart rate sensor (3) can be used to measure a SPO₂ signal. More particularly, one LED of the emitter 6 can emit in the red band of light (660 nanometers) and one in the infrared band of light (940 nanometers). Oxyhemoglobin absorbs infrared light while deoxyhemoglobin absorbs visible red light. The receiver 7 can detect the ratio of red/infrared absorption several hundred times per second. A preferred algorithm for calculating the absorption is derived from the Beer-Lambert Law, which determines the transmitted light from the incident light multiplied by the exponential of the negative of the product of the distance through the medium, the concentration of the solute and the extinction coefficient of the solute. The motion reference signal can be used to obtain a SPO₂ signal free from motion artifacts.

The PPG-based heart rate sensor (3) of the monitoring device (1) can also be used for the measurement of other parameters than the CV and SPO₂ signals, such as arterial blood pressure, breathing, hypovolemia, etc. Alternatively, the monitoring device (1) can also comprise additional sensors such as a temperature sensor for measuring the temperature on the user's head, or a sensor for measuring electrodermal activity, etc.; the additional sensors being preferably placed on the headband (2).

In yet another exemplary embodiment, the activity level reference signal and robust heart rate signal can be used in combination with the signal processing module 5 to control functionalities of the control device 9 such as, for example, the selection of a given music in function of the heart rate and/or steps frequency. In addition to the heart rate, the software can be configured to prompt the user with other data such as level of exercise intensity, type of exercise to be undertaken, etc.

In yet another exemplary embodiment, the robust heart rate signal is used as a supplementary parameter, for example, indicating the level of difficulty of the activity. For example, in a fitness activity the speed and/or duration of the activity can be adjusted in response to the heart rate signal. The heart rate signal and the activity level reference signal can also be used as parameters in the activity in order to obtain optimal benefit of the activity, for example, allowing the user to define his objectives such as losing weight or improving fitness.

The monitoring device 1 can then comprise a transfer device for transferring the heart rate and activity level reference signals produced by the heart rate sensor 3 and the motion sensor 4 to an external console, for example, associated with a running belt or a cycling apparatus. The robust heart rate signal and determined activity level of the user can be simply displayed on a screen of the console, or used in combination with the fitness activity or game or process.

### Reference numbers

- 1: monitoring device
- 2: headband
- 3: heart rate sensor
- 4: motion sensor
- 5: signal processing module
- 6: emitter
- 7: receiver
- 8: acquisition module
- 9: control device
- 10: cable
- 11: memory means
- 12: output device

## Claims

1. A monitoring device (1) worn by a user comprising a heart rate sensor (3) for delivering a sensor cardiovascular (CV) signal, a signal processing device (5) for correcting artifacts due to user's motion in said sensor CV signal, and a motion sensor (4) delivering a motion reference signal; the motion sensor (4) being **characterized by** having :
• a main function for determining and outputting a signal representing the level of activity of the user, and
• an auxiliary function for controlling said signal processing device (5).

2. The monitoring device (1) according to claim 1, where the motion reference signal delivered by the motion sensor (4) is exploited in the signal processing device (5) to provide a robust heart rate signal corrected for the movement artifacts from the sensor CV signal.

3. The monitoring device (1) according to one of claims 1 or 2, where
the motion reference signal delivered by the motion sensor (4) is exploited in the signal processing device (5) to provide an activity level reference signal representative of the level of activity of the user.

4. The monitoring device (1) according to any of the claims from 1 to 3, where
the robust heart rate signal is used in combination with the activity level reference signal to assess and monitor the fitness of the user or the efficacy of a sportive activity during an activity process.

5. The monitoring device (1) according to any of the claims from 1 to 4, where
the signal representing the level of activity corresponds to the speed of the user.

6. The monitoring device (1) according to any of the claims from 1 to 5, where
the heart rate sensor (3) and the motion sensor (4) are in a headband (2), worn on the user's head.

7. The monitoring device (1) according to any of the claims from 1 to 6, where
the motion sensor (4) is within the heart rate sensor 3.

8. The monitoring device (1) according to claims 6 or 7, where the signal processing module (5) is also in the headband (2).

9. The monitoring device (1) according to claims 6 or 7, where the signal processing module (5) is within an external control device (9).

10. The monitoring device (1) according to claim 9, where the external control device (9) is a media player or a handheld computing device or a wristwatch.

11. The monitoring device (1) according to any of the claims from 1 to 10, where
the heart rate sensor (3) is based on an electro-optic technique.

12. The monitoring device (1) according to claim 11, where the heart rate sensor (3) comprises a pair of infrared light emitting devices (LED) as emitter (6) and a photodiode as receiver (7).

13. The monitoring device (1) according to any of the claims from 1 to 12, where
the motion sensor (4) is a two-dimensional accelerometer able to provide at least an acceleration signal representative of the vertical acceleration.

14. The monitoring device (1) according to any of the claims from 1 to 13, where
the monitoring device (1) further comprises an output device (12) for outputting an indication of the robust heart rate signal and/or the determined activity level reference signal.

15. The monitoring device (1) according to any of the claims from 1 to 14, where
the monitoring device (1) further comprises the monitoring of oxygen saturation of arterial blood (SPO₂) by measuring a SPO₂ signal using the heart rate sensor (3).

16. The monitoring device (1) according to claim 15, where the motion reference signal is used to obtain a SPO₂ signal free from motion artifacts.

17. Method for processing a sensor cardiovascular (CV) signal using a motion sensor (4) available in a monitoring device (1) and having a main function for determining and outputting a signal representing the level of activity of a user, and an auxiliary function where a motion reference signal provided by the motion sensor (4) is used in a signal processing device (5) to determine a heart rate signal corrected for the movement artifacts from the sensor CV signal.

18. Method according to claim 17, where the processing of the sensor CV signal is aimed at correcting the sensor CV signal for motion artifacts and comprises the steps of:
a) detecting the sensor CV signal using the heart rate sensor (3);
b) providing a motion reference signal using a motion sensor (4) during the sensor CV signal detection period;
**characterized by**:
c) extracting a fundamental movement frequency from the motion reference signal;
d) enhancing the detected sensor CV signal with the fundamental movement frequency using linear or non-linear, model-based noise cancelling techniques;
e) extracting heart rate signals from the enhanced sensor CV signal;
f) assessing of reliability of extracted heart rate signals;
g) merging and/or discarding heart rate signals based on the reliability assessed in the previous step in order to obtain a reliable heart rate signal corrected for motion artifacts.

19. Computer carrier comprising program code portions to be executed by a signal processing device (5) in order to carry out the method of the claims 17 or 18 when said program is executed by said signal processing device (5).
